# EUROPÄISCHE PATENTSCHRIFT

(11) **EP 1 127 537 B1**
(45) Veröffentlichungstag und Bekanntmachung des Hinweises auf die Patenterteilung: **02.07.2008**
(21) Anmeldenummer: 01103563.1
(22) Anmeldetag: 20.02.2001
(51) Int. Cl.: A61B 5/00

(54) **Tragbarer Recorder zur Aufnahme und Übertragung eines mehrkanaligen EKG**
Portable recorder for recording and transmitting a multichannel ECG
Enregistreur portable pour l'enregistrement et la transmission d'un ECG à canaux multiples

(30) Priorität: 23.02.2000 DE 10008411
(43) Veröffentlichungstag der Anmeldung: 29.08.2001
(73) Patentinhaber: von Berg Medizingeräte GmbH, 08297 Zwönitz (DE)
(72) Erfinder: Scharner, Wilfried, Dr.-Ing., 08280 Aue (DE); Rentzsch, Michael, Dr.-Ing., 53359 Rheinbach (DE)
(74) Vertreter: Horn, Klaus

(56) Entgegenhaltungen:
- US-A- 5 012 411
- US-A- 5 228 450
- US-A- 5 544 661
- US-A- 5 678 562
- US-A- 5 941 829

## Beschreibung

Die Erfindung ist dem Gebiet der Medizintechnik zugehörig und betrifft das Messen, Aufzeichnen und Übertragen von bioelektrischen Signalen des Körpers oder von Teilen desselben zu diagnostischen Zwecken, insbesondere ein Verfahren zur Aufnahme und Übertragung eines mehrkanaligen EKG und eine Anordnung als tragbarer Recorder zur Durchführung des Verfahrens. Dieses Verfahren und diese Anordnung ist Teil eines telemedizinischen Systems EKG - Recorder - Mobiltelefon - Auswertezentrale, welcher das Kernstück einer mobilen EKG-Überwachung gefährdeter Patienten bildet.

Einrichtungen zur mobilen Patientenüberwachung sind hauptsächlich in Form von zwei sich unterscheidenden Gerätearten, mit den damit verbundenen sich unterscheidenden Verfahrensweisen bekannt.

Zur ersten Gerätegruppe gehören die Mobiltelefone. Als Repräsentanten dessen, sind z.B. die technischen Lösungen nach der EP 0 679 041 A2 und DE PS 197 07 681 C1 zu nennen. Die technische Lösung nach der EP 0 679 041 A2 ist lediglich dazu geeignet, die Ortung des Patienten mit seinen Positionsdaten sicherzustellen. Bei der technischen Lösung nach der v.g. DE PS werden die ausgewählten medizinischen Daten mittels spezieller Kontakte am Mobiltelefon, die am Körper des Patienten anliegen oder mittels Leitungen dorthin verbracht werden, gemessen und in das Funknetz übertragen, wonach die Ergreifung sofortiger Hilfsmaßnahmen möglich ist. Über die reine Positionsübermittlung des Patienten nach v.g. EP-Schrift hinausgehend werden hierbei, im Gegensatz zur v.g. EP-Lösung, die augenblicklichen Daten zur Patientenidentifikation und/oder der EKG-Signal-Aufnahme sofort übermittelt. Eine Offenbarung dessen, daß ein solches vorgenanntes Mobiltelefon selbsttätig überwacht und selbsttätig bei einem medizinisch signifikanten Ereignis (Event) die ermittelten Daten in das Funknetz übermittelt, ist nicht nachweisbar, insbesondere aber auch deswegen nicht zu vermuten, da eine vom Patienten zu betätigenden Notruftaste am Gerät beschrieben wird, die der Patient selbst bei bedrohlichen Situationen betätigen kann bzw. muß. Als nachteilig bei diesen v.g. technischen Lösungen ist zu konstatieren, daß keine Speicherung und Aufzeichnung großer Mengen relevanter Daten bei einer ununterbrochen langen, mehrtägigen, mehrwöchigen bzw. mehrmonatigem Überwachungsmaßnahme möglich ist. Es wird auch keine Betriebsmöglichkeit der Übertragung einer gespeicherten und aufgezeichneten Datenmenge an einen "zentralen" Computer zur Auswertung der Zeitverläufe der relevanten Daten offenbart. Zur Übertragung kommen bei vorgenannten Lösungen also nur die Daten der normalen Augenblicksituation oder bei einem speziellen augenblicklichen Ereignis (Event) und nicht die Vorgeschichte zum Ereignis und ihre nachfolgende Auswertung. Darüber hinaus unterscheiden sich diese Funktelefone wesentlich von den üblichen, zum Telefonieren vorgesehenen, da sie Spezialgeräte mit spezieller Verwendungstechnik und meist kaum für das übliche Telefonieren vorgesehen bzw. geeignet sind.

Zur zweiten Gerätegruppe gehören die Recorder.

Es sind mehrere Arten von am Körper zu tragenden Recordern bekannt geworden.

Sogenannte Langzeit-EKG-Recorder zeichnen das EKG des Patienten im Regelfall kontinuierlich, meist über 24 Stunden, mittels elektronischer Speicher auf. Diese Recorder mit integriertem Datenträger werden danach zum Arzt gebracht. Die Daten werden ausgelesen bzw. entnommen und später ausgewertet.

Mit sogenannten Eventrecordern kann ein kurzer EKG-Abschnitt, meist im Sekundenbereich, beim Auftreten eines kritischen Herzereignisses oder in festgelegten Abständen aufgenommen und Übertragen werden. Diese Übertragung erfolgt transtelefonisch mit einem Festnetztelefon in einer aufwendigen Prozedur. Das gespeicherte EKG wird tonfrequenzmoduliert und erlaubt so eine Telefonübertragung in eine Auswertezentrale.

Bei sogenannten Eventrecordern mit Loopfunktion wird mittels am Körper angeklebter Elektroden fortlaufend ein EKG gespeichert und nach Sekunden- oder Minutenfrist fortlaufend überschrieben. Bei Auslösung eines Events ist somit die Vorgeschichte zum Ereignis gespeichert. Die Übertragung des EKG zur Zentrale erfolgt dann wie bei den vorherigen Eventrecordern.

Neuerdings sind implantierbare Recorder bekannt geworden. Der interne EKG-Speicher erlaubt eine Speicherung bis zu ca. einer Stunde. Das EKG kann extern ausgelesen werden.

Die nachteiligen technischen Wirkungen dieser vorgenannten technischen Lösungen in der Form der Anordnungs- wie auch der Verfahrensmerkmale bestehen insbesondere in folgendem.

Die Langzeit-EKG-Recorder erlauben eine Analyse des EKG, und somit das Erkennen von Herzrythmusstörungen, erst nach der Aufzeichnung. Die Kontrolle des Patienten erreicht nur 24 Std. bzw. 48 Std. und die Kontrollergebnisse werden aber auch erst nach einer weiteren erheblichen Zeitverzögerung erkennbar. Es ist also nur eine Diagnose, auch als "Diagnose danach" bezeichnet, möglich, die - auf seltene Ereignisse bezogen - eine sehr begrenzte Trefferwahrscheinlichkeit hat und den Verlauf z. B. bei medikamentöser Therapie praktisch nicht ermöglicht.

Den Nachteil der "Diagnose danach" sollen Eventrecorder weitgehend vermeiden. Es werden EKG-Ereignisse bzw. -Abschnitte aufgezeichnet. Diese werden aber mit einer komplizierten und aufwendigen Prozedur, die zwangsläufig Qualitätsverluste bewirkt und ein Festnetztelefon mit Tonfrequenzmodulierung erfordert, an eine Auswertezentrale übertragen. Das ganze ist sehr umständlich handhabbar, was eine Anwendung in der besonders gefährdeten Altersgruppe stark einschränkt, wenn nicht gar unmöglich macht. Weiterhin ist auch eine echte Übertragung im Notfall kaum möglich. Die Mobilität des Patienten ist durch das erforderliche Festnetztelefeon eingeschränkt. Ein Hilfe einer zweiten Personen ist auf Grund der umständlichen Prozedur i.d.R. erforderlich und im Streßfall sehr wahrscheinlich fehlerbehaftet. Hinzu kommt bei der ersten Gruppe der Recorder, daß ein kurzes Ereignis nie aufgezeichnet wird, da das Event-Auslösen durch den Patienten selbst erkannt werden muß und zwangsläufig erst danach erfolgt. Der diagnostische Wert ist somit sehr eingeschränkt.

Implantierte Recorder erfordern zwar keinen Applikationsaufwand, verlangen aber eine Implantation und müssen nach etwa 1 Jahr ausgetauscht werden. Eine Implantation eines Gerätes zu diagnostischen Zwecken kann nur einer kleinen Anzahl sehr gefährdeter Patienten zugemutet werden. Die Kosten hierfür sind bekanntermaße sehr hoch. Implantierte Geräte sind auf Grund ihres Aufbaus nur bedingt als Eventrecorder wirksam.

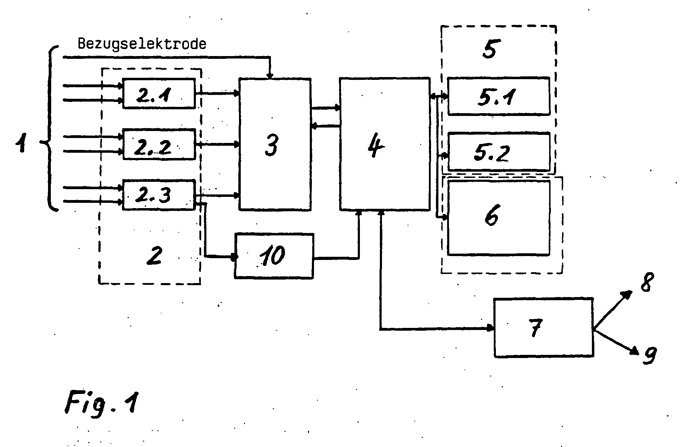

Die technische Lösung nach DE OS 198 48 229 A1 beschreibt eine Vorrichtung zur Aufzeichnung und Übertragung von digitalisierten medizinischen Daten zur On-line-Überwachung eines Patienten, die eine Datenerfassungskomponente (A) und eine "Handy-Einheit" (B) zu einem Gerät verbunden beinhaltet. Hierbei werden die EKG-Daten vom Analog-Digital-Wandler direkt zum RAM-Speicher geleitet und von dort wiederum direkt zur Handy-Einheit. Damit ist eine Behandlung und/oder Steuerung der Daten, zum Beispiel im Sinne einer Datenkompression, nicht möglich, wie auch aus den beiden einzigen Figuren der Schrift, siehe Pfeilverlauf für den Signalverlauf, erkennbar ist. Die technische Funktion ist gemäß Offenbarungsgehalt der Schrift in Frage zu stellen, da es wohl zu nichts führt, die A-D-gewandelten Signale 0,1 zu speichern, ohne eine durch einen Kontroller geordnete und gemanagte Datenmenge systemgerecht und geordnet zu speichern, um sie danach einer weiteren Verarbeitung und/oder Verwendung zuzuführen. Darüber hinaus läßt es die Figur 1, wie auch die Figur 2, der DE OS 198 48 229 nicht zu den Speicherinhalt direkt auf einen Computer (PC) auszulesen. Die in dieser DE OS im Hauptanspuch benannten und beanspruchten Merkmale stützen diese Beurteilung, da die dort genannten Anordnungsmerkmale die Verfahrensmerkmale " ...die analogen Aufzeichnungsdaten der Elektroden im Wandler digitalisieren, im elektronischen Speicher speichern und von dort von der Handy-Einheit über ein Funknetz weitergegeben ..." auslösen sollen. Im Beschreibungsteil in Spalte 2, Zeile 39 bis 41 wird im Gegensatz zum eben Dargestellten ein Lesen des Speichers beschrieben, was aber gemäß des Offenbarungsgehaltes dieser Schrift mit dieser Anordnung nicht möglich ist. Ein weiterer Nachteil der technischen Lösung nach der DE OS 198 48 229 stellt sich darin dar, daß mit den dort beschriebenen Schwellenschaltern nur einfache Rhythmusstörungen erkannt werden können und daß es nicht möglich ist kritische Arrhythmien zu erkennen, wodurch der medizinische Wert bzw. die erforderliche Sicherheit für den Patienten in Frage gestellt ist.

Nachteilig ist letztendlich auch, daß Datenerfassungs- und Aufzeichnungseinheit sowie Handy-Einheit in einem Gerät vereinigt sind, ist es doch viel universeller und auch kostengünstiger mit einer Datenerfassungs- und Aufzeichnungseinheit und einem handelsüblichen Handy zu arbeiten als letzteres vielleicht doppelt vorliegend zu haben, weil die speziellere und im übrigen nicht nutzbare Handy-Einheit schon in der technischen Lösung nach DE OS 198 48 229 enthalten ist.

Darüber hinaus sind alle diese Einrichtungen mit ihren zugehörigen Verfahrensabläufen nicht in der Lage EKG's in beliebiger Länge aufzunehmen, zu speichern und bei Bedarf mit Hilfe eines beliebigen, handelsüblichen Standard-Funktelefones (Handy) an eine Zentrale zu übertragen, um dort die sofortige Auswertung, in der Regel in Computersystemen, zu ermöglichen.

Aus US-A-5 678 562 ist ein tragbarer physiologischer Monitor bekannt, der einen herausnehmbaren Plattenspeicher aufweist. Der tragbare Monitor kann ein Modem zur Kommunikation mit einem entfernt aufgestellten Computer aufweisen, wobei ein Handynetz verwendet wird. Es können ausgewählte Abschnitte physiologischer Daten, z. Bsp. EKG-Daten, oder Analysenergebnisse dieser Daten übertragen werden.

US-A-5 012 411 offenbart ein tragbares, von einem Mikroprozessor kontrolliertes Gerät, das EKG-Daten aufnimt und speichert, sowie auf einem EKG-Signal Schrittmacherimpulse detektiert. Das Gerät verfügt über ein Modem, um über eine Telefonleitung mit einem Computer zu kommunizieren.

Von den Mängeln und deren Ursachen des oben dargestellten Standes der Technik ausgehend, liegt der Erfindung die Aufgabe zugrunde, eine Verfahrensweise und eine Anordnung zu schaffen, die es ermöglichen, eine automatisierte Übertragung des aufgezeichneten EKG in beliebiger Länge, wie auch eine automatische Übertragungsauslösung bei kritischen Arrhythmien mit einem (handelsüblichen) Standard-Mobiltelefon vorzunehmen und damit einen Komplex "EKG-Übertragung" in voller Mobilfunkleistung zu bilden. Aufgabe der Erfindung soll es weiterhin sein, eine so hohe Speicherkapazität zu realisieren, daß die Anordnung diskontinuierliche EKG-Überwachung über einen langen Zeitraum von Tagen, Wochen bis Monaten und kontinuierliche EKG-Überwachung über 48 Stunden wahlweise ermöglicht, wobei auch eine Entnahme des Speichermediums möglich sein soll. Eine volle Mobilität des Patienten bei EKG-Aufnahmen und Übertragungen soll gesichert sein.

Diese Aufgabe wird für ein gattungsgemäßes Verfahren und eine gattungsgemäße Anordnung erfindungsgemäß durch die in den Ansprüchen 1 bis 8 angegebenen Merkmale gelöst.

Es besteht somit eine Anordnung, die sich als ein tragbarer Recorder mit den Hauptbaugruppen mehrkanaliger Verstärker, A-D-Wandler, Mikrokontroller, Speicherelemente RAM und flash-ROM, einer herausnehmbaren flashcard, Schrittmacherkontroller und Schnittstelle darstellt. Bewußt wurde eine Mobil-Telefoneinheit innerhalb dieser erfindungsgemäßen Anordnung nicht vorgesehen, da damit unnützer Aufwand in teilweise beschränkender Spezialfunktionalität verbunden ist. Ziel der Erfindung war es ja auch, die universellen, handelsüblichen und sich bereits in Millionen-Stückzahlen in der Bevölkerung durchgesetzten Mobiltelefone (Handys) in die Datenkommunikation erfindungsgemäß einzubringen. Bisher bekannte, moderne jüngere Technik zur mobilen Patientenüberwachung beachteten diesen wirtschaftlich sehr bedeutenden Gedanken nicht und gingen stets davon aus, die Mobilfunk-Technik in abgewandelter Weise eignungsfähig in ein solches medizinisches Überwachungssystem einzubinden. Die Vorteile der Trennung von Datenerfassung und -aufzeichnung von der Mobiltelefon-Einheit liegen förmlich auf der Hand. Neben einem einfacheren Aufbau und weniger Arbeits-, Zeit-, Material- und Kosten-Aufwand bei der Herstellung, dem Vertrieb und im Einsatz (hier insbesondere das Gewicht), da ansonsten "doppelt", ist jedes Mobiltelefon einsetzbar, was vielfach ohnehin schon vorhanden ist. Natürlich wird sich damit diese erfindungsgemäße technische Lösung immer mit einem Mobiltelefon darstellen. Die Recorderbaugruppen ermöglichen die Datenkommunikation mit dem Mobiltelefon in der Weise, daß ein definierter Telefonanruf erfolgt, wenn dies die Recorder-Anordnung dem Mobiltelefon signalisiert, um damit und danach die EKG-Daten mit den Patientendaten im geeigneten Datenprotokoll übertragen werden können, sowie eine Daten- und Übertragungssicherung erfolgt. Mittels der sich im Signal- bzw. Datenverlauf vom Stand der Technik gemäß DE OS 198 48 229 erfindungswesentlich abhebenden Anordnung bzw. Datenbearbeitung des bzw. durch den Mikrokontroller und durch ein spezielles Datenprotokoll ist es überhaupt erst möglich, eine mehrkanalige EKG-Übertragung mittels einer Datenkomprimierung der einzelnen Kanäle durchzuführen, was sich auch in kürzeren Übertragungszeiten niederschlägt. Der v.g. nächst gelegene Stand der Technik kennt diese Mittel-Wirkungs-Beziehung nicht. Dort gelangen die EKG-Daten vom A-D-Wandler direkt zum RAM-Speicher und von da wiederum direkt zur Handy-unit. Damit ist eine datenkomprimierende Behandlung/Steuerung dieser Daten nicht möglich. Im Vergleich zur Anordnung des v.g. nächst gelegenen Standes der Technik ergeben sich durch diese Erfindung vorteilhafte Wirkungen derart, daß bei der hier beschriebenen erfindungsgemäßen Lösung die Daten vor dem Senden zum Mobiltelefon durch den Mikrokontroller eine Komprimierung erfahren, was die Übertragungszeit etwa um den Faktor 5 gegenüber der nicht komprimierten Übertragung verkürzt, daß diese erfindungsgemäße technische Lehre eine theoretisch unbegrenzte Zahl einzelner EKG-Kanäle übertragen kann, auch wenn die Datenmengen infolge unterschiedlicher Kompressionsmöglichkeiten in den einzelnen Kanälen verschieden voneinander sind. Im Gegensatz zum nächst gelegenen Stand der Technik verwendet diese technische Lehre erfindungsgemäß eine Speicherkarte in Flash-Technologie (flashcard) zur Aufzeichnung. Diese Speichermethode erlaubt auch eine 24- oder 48-stündige Aufzeichnung in Verbindung mit der telefonischen Übertragung bzw. sie gestattet die Aufzeichnung einer sehr großen Zahl von einzelnen Events kurzer Dauer. Es ist also auch in einem zweiten Modus die "Nur Speicherung" des kurz- oder langzeitigen EKGs möglich, so daß eine neuartige Betriebsart der Methoden Langzeit-EKG und Eventrecording geschaffen wurde. Weiterhin erlaubt die technische Lehre mit einer flashcard die nichtflüchtige Speicherung der Daten auch bei Batteriewechsel oder vollkommener Erschöpfung der Batterie. Somit können aufgezeichnete Daten zu einem beliebigen späteren Zeitpunkt über das Mobiltelefon übertragen werden und auch ein zwischenzeitlicher Batteriewechsel führt nicht zum Datenverlust. Schließlich kann die erfindungsgemäß eingesetzte flashcard mit den gespeicherten Daten aus dem Recorder entnommen werden und an anderer Stelle in einen PC eingelesen werden. Das erweitert die Anwendungsmöglichkeiten bei einer Speicherung von großen Datenmengen. Das Auslesen der Daten über die serielle Schnittstelle ist nicht zwingend notwendig.

Die erfindungsgemäße technische Lösung hat also den Vorteil von 3 Methoden, somit:
- Eventrecording im Loop-Modus einschließlich Speicherung der Vorgeschichte mit der Möglichkeit der Sofortübertragung über Mobiltelefon im Notfall,
- Eventspeicherung zur nachträglichen Auswertung, sowie eine
- komplette mehrtägige kontinuierliche Langzeit-EKG-Speicherung.

Für Eventrecording ist nur die Eventauslösung mit einer Taste notwendig; die weiteren Verfahrensabläufe erfolgen vollautomatisch.

Eine automatische Übertragung des EKG bei kritischen Arrhythmien erfolgt sicher durch eine spezielle Software. Die im Stand der Technik beschriebenen Schwellenschalter können nur einfache Rhythmusstörungen erkennen, was den medizinischen Wert bzw. die erforderliche Sicherheit für den Patienten in Frage stellt.

Anhand eines Ausführungsbeispieles, welches in Fig. 1 dargestellt ist, wird die Erfindung noch kurz näher erläutert.

Die Fig. 1 zeigt das Blockschaltbild, so wie sich die Erfindung darstellen kann. Von den aufgenommenen analogen Signalen 1 werden drei EKG-Signale einem dreikanaligen Verstärker 2 mit seinen drei Kanälen 2.1, 2.2, und 2.3 zugeleitet. Die digitalisierten Signale aus den drei Kanälen Kanälen 2.1, 2.2, und 2.3 werden dem Mikroprozessor 4 zugeleitet, der seinerseits auf den A-D-Wandler 3 bei Bedarf und Notwendigkeit Einfluß nehmen kann. Aus einem EKG-Kanal, z.B. dem Kanal 2.3, ist zudem eine Datenleitung auf den Schrittmacherkontroller 10 geschaltet, der bei einem Schrittmacher-EKG die Schrittmacherimpulse detektiert und das EKG am detektierten Zeitpunkt markiert und dem Mikrokontroller 4 zuführt. In den dem Mikroprozessor 4 parallel nachgeordneten Speichereinrichtungen 5 und 6 werden die bearbeiteten Daten entsprechend ihrem weiteren Verwendungszweck abgespeichert, wovon sie vom Mikrokontroller 4 bedarfsweise abgerufen und gegebenenfalls weiter bzw. erneut bearbeitet werden können. Die flashcard 6 ist aus der Recorderanordnung herausnehmbar angeordnet, damit diese dort gespeicherten Daten an anderer Stelle in einen PC eingelesen werden können und vermittels dessen. bearbeitet und ausgewertet werden können. Dies erweitert die Anwendungsmöglichkeiten bei einer Speicherung großer Datenmengen und das Auslesen ist nicht zwingend über die serielle Schnittstelle notwendig. Die vorgesehene serielle Schnittstelle 7, die an den Mikrokontroller 4 angeschaltet ist übergibt die Daten wahlweise einem PC 9 oder dem handelsüblichen Mobiltelefon 8. Der entgegengesetzte Signalfluß vom Mobiltelefon 8 oder dem PC 9 zum Mikrokontroller 4 ist bei Bedarf auch möglich.

Vor der Inbetriebnahme zur EKG-Überwachung wird der Recorder über die serielle Schnittstelle mit Patienten- und Übertragungsdaten konfiguriert.

In der Auswertezentrale wird der Patient decodiert und das EKG in der Datenbank zugeordnet. Damit ist eine absolut sichere Identifizierung gegeben. Ein empfangenes EKG wird signalisiert, so daß eine sofortige Auswertung möglich ist und die entsprechenden Maßnahmen eingeleitet werden können, z.B. ein Rückruf zum Patienten über das Mobiltelefon oder die Alarmierung eines medizinischen Notdienstes. Ist eine Übertragung des EKG durch unzureichende Funkübertragungsmöglichkeiten nicht gesichert, so erfolgen Wahlwiederholungen. Erfolgte auch danach keine Übertragung, wird das gespeicherte EKG beim nächsten Event mit übertragen.

## Patentansprüche

1. Verfahren zur Aufnahme und Übertragung eines mehrkanaligen EKG mit einem Recorder als Teil eines telemedizinischen Systems Recorder - Übertragungselement - Auswertezentrale, bei dem EKG-Daten eines Patienten mittels des mobilen am Körper eines Patienten tragbar angeordneten Recorders permanent abgenommen, digitalisiert, gespeichert und per Funk an eine Auswertezentrale zur näherungsweise zeitgleichen Auswertung bzw. Überwachung übertragen werden,
wobei nach der Abnahme der EKG-Daten die EKG-Signale (1) mittels eines mehrkanaligen Verstärkers (2) und eines anschließenden A-D-Wandlers (3) aufbereitet werden; diese so aufbereiteten EKG-Signale (1) einem Mikrokontroller (4) zur Bearbeitung zugeführt werden, welcher bedarfsweise den A-D-Wandler abfragt; die vom Mikrokontroller (4) bearbeiteten EKG-Signale auf einer Speicherkombination (5) und zeitbezogen auf einer flashcard (6) gespeichert werden, wobei ein Rückfluß der Daten von der Speicherkombination (5) und der flashcard (6) zum Mikrokontroller vorgesehen ist; der Mikrokontroller eine serielle Schnittstelle (7) für eine Recorderkonfiguration und das Auslesen des aufbereiteten EKG mittel einer speziellen Software so steuert, daß einerseits eine Übertragung zu einem Standard-Mobiltelefon (8) und andererseits das Auslesen direkt zu einem Computer (9) vorgenommen werden kann; sowie eines der aus dem mehrkanaligen Verstärker (2) kommenden EKG-Signale einem Schrittmacherkontroller (10) zugeführt wird, der bei einem Schrittmacher-EKG die Schrittmacherimpulse detektiert, das EKG am detektierten Zeitpunkt markiert und dem Mikrokontroller (4) zuführt.

2. Verfahren nach Anspruch 1, **dadurch gekennzeichnet, daß** nach einer Übertragung/Speicherung des mehrkanaligen EKG in einer Auswertezentrale die Auswertung dieses EKG über bekannte Auswerteprogramme erfolgt.

3. Verfahren nach Anspruch 1, **dadurch gekennzeichnet, daß** im Mikrokontroller (4) des Recorders durch eine spezielle artefaktgesicherte Software eine permanente Überwachung nach kritischen Arrhythmien, die eine sofortige Beurteilung erfordern, erfolgt und nach Erkennung einer solchen Arrhythmie automatisch das aktuelle EKG sowie das EKG vor dieser Störung per Mobiltelefon zur Zentrale übertren wird.

4. Verfahren nach Anspruch 1, **dadurch gekennzeichnet, daß** mit Hilfe einer speziellen Software bei Übertragung per Mobiltelefon automatisch das Telefon eingeschaltet und nach Auslösen eines Events automatisch eine gespeicherte Ruf-Nr. zur Zentrale gewählt wird.

5. Verfahren nach Anspruch 1 bis 4, **dadurch gekennzeichnet, daß** mit der gleichen Verfahrensweise gemäß v.g. Verfahrensmerkmale und der gleichen Anordnung Eventrecording und kontinuierliche Langzeitüberwachung durchgeführt wird.

6. Anordnung als tragbarer Recorder zur Durchführung des Verfahrens gemäß einem der Ansprüche 1-5 beinhaltend eine A-D-Wandler-Einheit, eine Speichereinheit und einen Mikrokontroller, wobei die EKG-Signale (1) auf einen mehrkanaligen Verstärker (2) mit den Kanälen (2.1, 2.2, 2.3) geschaltet sind, und ein oder mehrere der EKG-Signale (1) auf einen dem Verstärker (2) nachfolgend angeordneten A-D-Wandler (3) auflaufen; dem Verstärker (2) der A-D-Wandler (3) sowie ein Schrittmacherkontroller (10), zueinander parallel angeordnet, nachgeschaltet sind und diese gemeinsam auf einen Mikrokontroller (4) aufgeschaltet sind; der Mikrokontroller (4) auf den davorliegenden A-D-Wandler (2) einflußnehmend konfiguriert ist; dem Mikrokontroller (4), wechselseitig einflußnehmend, eine Speicherkombination (5) und eine flashcard (6) sowie eine serielle Schnittstelle (7) zugeordnet sind, wobei letztere den Zugang zum Standard-Mobiltelefon (8) und zum Computer (9) bildet.

7. Anordnung nach Anspruch 6, **dadurch gekennzeichnet, daß** die Speicherkombination (5) aus wenigstens einem RAM (5.1) und einem flash-ROM (5.2) gebildet ist.

8. Anordnung nach Anspruch 6, **dadurch gekennzeichnet, daß** die flashcard (6) herausnehmbar in der Recorderanordnung angeordnet ist.

## Claims

1. Method to record and transmit a multichannel ECG with a recorder as part of a telemedicine system recorder-transmission element-evaluation centre, in which ECG data of a patient are continuously traced, digitised, stored and transmitted by radio signal to an evaluation centre by way of a mobile recorder arranged to be carried on the body of the patient for approximately immediate evaluation or monitoring,
wherein after tracing of the ECG data the ECG signals (1) are conditioned by way of a multichannel amplifier (2) and a subsequent A/D converter (3); these accordingly conditioned ECG signals (1) are passed for processing to a microcontroller (4) which interrogates the A/D converter as required; the ECG signals processed by the microcontroller (4) are stored in a memory combination (5) and synchronously on a flashcard (6), with provision for a return data flow from the memory combination (5) and the flashcard (6) to the microcontroller; the microcontroller controls a serial interface (7) for recorder configuration and to control the reading out of the conditioned ECG with a special software in such a manner that on the one hand transmission to a standard mobile telephone (8) and on the other hand direct output to a computer (9) can be realised; and one of the ECG signals coming from the multichannel amplifier (2) is passed to a pacemaker controller (10), which detects the pacemaker pulses for a pacemaker ECG, marks the ECG at the detected times and passes the same to the microcontroller (4).

2. Method according to claim 1, **characterised in that** after transmission/saving of the multichannel ECG the evaluation of this ECG is performed in an evaluation centre with known evaluation programs.

3. Method according to claim 1, **characterised in that** a special artefact-safe software in the microcontroller (4) of the recorder performs permanent monitoring for critical arrhythmia requiring immediate assessment and after detecting any such arrhythmia automatically transmits the current ECG and the ECG before this irregularity to the evaluation centre via mobile telephone.

4. Method according to claim 1, **characterised in that** the telephone is switched on automatically by way of a special software in case of transmission by mobile telephone and a saved evaluation centre telephone number is called automatically when an event is triggered.

5. Method according to claims 1 to 4, **characterised in that** the same procedures as those described in the aforementioned claims and the same arrangement are used to perform event recording and continuous long-term monitoring.

6. Arrangement as a portable recorder to implement a method as described in one of the claims 1 to 5, comprising an A/D converter unit, a data storage unit and a microcontroller, wherein the ECG signals (1) are delivered to a multichannel amplifier (2) with the channels (2.1, 2.2, 2.3) and one or several of the ECG signals (1) are led to an A/D converter (3) arranged subsequent to the amplifier (2); the A/D converter (3) and a pacemaker controller (10) arranged parallel to each other are arranged after the amplifier (2) and connected commonly to a microcontroller (4); the microcontroller (4) is configured to exert influence on the preceding A/D converter (3); a memory combination (5) and a flashcard (6) and furthermore a serial interface (7) are assigned to the microcontroller (4) such as to permit reciprocal influence, with the serial interface (7) providing for access to the standard mobile phone (8) and to the computer (9).

7. Arrangement according to claim 6, **characterised in that** the memory combination (5) comprises at least one RAM element (5.1) and one flash ROM element (5.2).

8. Arrangement according to claim 6, **characterised in that** the flashcard (6) is arranged such that it can be removed from the recorder arrangement.

## Revendications

1. Procédé pour l'enregistrement et la transmission d'un ECG à plusieurs canaux avec un enregistreur comme partie d'un système télé-médical enregistreur - élément de transmission - centrale d'évaluation, à l'occasion de quoi les données ECG du patient sont prises en permanence, numérisées, enregistrées au moyen d'un enregistreur mobile, disposé de façon portable sur le corps du patient, et sont transmises via radio à une centrale d'évaluation pour l'évaluation et/ou surveillance approximativement simultanée,
à l'occasion de quoi, après la prise des données ECG, les signaux ECG (1) sont transformés à l'aide d'un amplificateur à plusieurs canaux (2) et d'un convertisseur AD consécutif (3) ; ces signaux ECG (1) ainsi transformés sont amenés vers un micro-contrôleur (4) pour traitement, lequel interroge le convertisseur AD en cas de besoin ; les signaux ECG, traités par le micro-contrôleur (4), sont enregistrés sur une combinaison mémoire (5) et rapportés au temps sur une carte flash (6), à l'occasion de quoi un reflux des données de la combinaison mémoire (5) et de la carte flash (6) vers le micro-contrôle est prévu ; le micro-contrôleur commande une interface sérielle (7) pour une configuration d'enregistreur et l'extraction de l'ECG ainsi préparé au moyen d'un logiciel adapté de telle façon, que d'une part on peut procéder à une transmission vers un téléphone portable standard (8) et d'autre part l'extraction peut s'effectuer directement vers un ordinateur (9) ; ainsi que l'un des signaux ECG de l'amplificateur à plusieurs canaux (2) est transféré à un contrôleur de pacemaker (10), qui détecte les impulsion de pacemaker dans un ECG de pacemaker, qui marque l'ECG au moment détecté et le transfère au micro-contrôleur (4).

2. Procédé d'après la revendication 1, **caractérisé par le fait, qu'**après une transmission/sauvegarde de l'ECG à plusieurs canaux dans une centrale d'évaluation, l'évaluation de cet ECG s'effectue via des programmes d'analyse connus.

3. Procédé après la revendication 1, **caractérisé par le fait, que** dans le micro-contrôleur (4) de l'enregistreur, via un logiciel particulier, sécurisé contre les parasites, s'effectue une surveillance continue d'arythmies critiques nécessitant une évaluation immédiate et après la reconnaissance d'une telle arythmie l'ECG actuel ainsi que l'ECG d'avant la perturbation soient transmis automatiquement vers la centrale.

4. Procédé d'après la revendication 1, **caractérisé par le fait, qu'**à l'aide d'un logiciel particulier lors de la transmission par téléphone portable le téléphone est connecté automatiquement et après le déclenchement d'un événement un numéro d'appel enregistré vers la centrale est composé automatiquement.

5. Procédé d'après la revendication 1 à 4, **caractérisé par le fait, qu'**avec le même mode opératoire énuméré ci-dessus et la même configuration on procède à l'enregistrement des événements et à une surveillance continue de longue durée.

6. Configuration comme enregistreur portable pour réaliser le procédé selon l'une des revendications 1-5, qui contient une unité de convertisseur AD, une unité de mémoire et un micro-contrôleur, à l'occasion de quoi les signaux ECG sont commutés (1) sur un amplificateur à plusieurs canaux (2) avec les canaux (2.1, 2.2, 2.3), et un ou plusieurs des signaux ECG (1) arrivent sur un convertisseur AD (3), disposé derrière l'amplificateur (2) ; ils sont placés après l'amplificateur (2), le convertisseur AD (3) ainsi qu'un contrôleur de pacemaker (10), qui sont disposés entre eux parallèlement, et ceux-ci ensemble sont commutés sur un micro-contrôleur (4) ; le microcontrôleur (4) étant configuré pour agir sur le convertisseur AD (2) situé en amont ; le microcontrôleur (4) interagissant avec une unité de mémoire (5) et une flashcard (6) ainsi qu'une interface sérielle (7), étant entendu que la dernière constitue l'accès au téléphone portable standard (8) et à l'ordinateur (9).

7. Disposition d'après la revendication 6, **caractérisé par le fait, que** la combinaison mémoire (5) est constituée d'au moins un RAM (5.1) et un flash-ROM (5.2).

8. Disposition d'après la revendication 6, **caractérisé par le fait, que** la carte flash (6) est disposée de façon amovible dans l'ensemble de l'enregistreur.
